# EUROPEAN PATENT APPLICATION

(11) **EP 4 796 074 A1**
(43) Date of publication of application: **26.08.2026**
(21) Application number: 26191544.1
(22) Date of filing: 29.08.2024
(51) Int. Cl.: A61C 9/00, A61B 5/00

(54) **INTRAORAL SCANNER SYSTEM WITH A CORRECTION FEEDBACK SIGNAL AND METHOD FOR GENERATING A CORRECTION FEEDBACK SIGNAL**

(30) Priority: 29.08.2023 DK PA202370444
(62) Document number(s) of the earlier application(s) in accordance with Art. 76 EPC: 24197152.2 / 4 516 263
(71) Applicant: 3Shape A/S, 1060 Copenhagen K (DK)
(72) Inventor: HOFFGAARD, Simon, 1060 Copenhagen K (DK)
(74) Representative: Zacco Denmark A/S

(57) **Abstract**

An intraoral scanner system (100) configured to display a correction feedback signal during a scan session is disclosed. The intraoral scanner system (100) comprises a handheld intraoral scanner (101) configured to obtain light information reflected from a dental object (108) inside an oral cavity through a field-of-view (105) of the handheld intraoral scanner (101), one or more processors configured to process the light information and to generate a digital 3D model (103) of the dental object (108) based on the processed light information, and a graphical user interface (102) configured to display the digital 3D model (103). The one or more processors are configured to display in real-time and on the graphical user interface (102) a field-of-view frame (104) representing a position of the field-of-view (105) of the handheld intraoral scanner (101) and generate a correction feedback signal that corresponds to the field-of-view frame (104), and wherein the correction feedback signal includes a suggested correction of the position of the field-of-view (105) of the handheld intraoral scanner (101).

## Description

### TECHNICALD FIELD

The disclosure relates to an intraoral scanner system with a correction feedback signal that guides a user towards a suggested scan area in an oral cavity. In particular, the disclosure relates to an intraoral scanner system comprising one or more processors configured to display, on a graphical user interface, a field-of-view frame displaying an actual field-of-view of a scan tip of a handheld intraoral scanner, and further configured to display the correction feedback signal.

### BACKGROUND

When scanning an intraoral cavity of a patient using a handheld intraoral scanner, a user, for example a dentist, may employ non-optimal scan methods. Thus it may occur that the user performing the scan misses certain regions of the intraoral cavity, resulting in a lack of light information received by the handheld intraoral scanners from those certain regions. This may occur, for example due to lack of experience in intraoral scanning. As a result, generation of a digital 3D model may be interrupted or an incomplete digital 3D model of the intraoral cavity may be created. The user may not follow an optimal scan path allowing for receiving minimum amount of light information sufficient for generating the digital 3D model of the intraoral cavity of a desired quality. Furthermore, during the scanning, the intraoral scanner system may lose registration of obtained light information, resulting in the user needing to navigate the handheld intraoral system to a position where the registration has been lost, and continuing the scanning from that position. Overall, the scanning process can be severely slowed down and obstructed. Therefore, there is a clear need for user guidance during the scanning process to improve efficiency and generate digital 3D models of desired quality.

Document US 9 724 177 B2 discloses methods and systems for scanning an intraoral cavity of a patient which provide capturing one or more viewfinder images of the intraoral cavity and scanning the intraoral cavity with an intraoral scanner to generate one or more topography scans of the intraoral cavity. The one or more topography scans may correspond to the one or more viewfinder images. The methods and systems further provide capturing a viewfinder image of a portion of the intraoral cavity, the viewfinder image overlapping with the one or more viewfinder images. An area of overlap of the viewfinder image with the one or more viewfinder images can be determined. One or more indicators of the area of overlap can be displayed on one or more locations of a display in order to provide guidance for positioning a field of view of the intraoral scanner.

### SUMMARY

An aspect of the present disclosure is to provide an intraoral scanner system that may provide assistance to its user by generating and displaying a correction feedback signal during a scan session.

According to an embodiment, the intraoral scanner system may be configured to display the correction feedback signal during the scan session, wherein the intraoral scanner system may comprise:
- a handheld intraoral scanner configured to obtain light information reflected from a dental object inside an oral cavity through a field-of-view of the handheld intraoral scanner;
- one or more processors configured to process the light information and to generate a digital 3D model of the dental object based on the processed light information; and
- a graphical user interface configured to display the digital 3D model,
wherein the one or more processors may be configured to:
- display in real-time and on the graphical user interface a field-of-view frame representing a position of the field-of-view of the handheld intraoral scanner; and
- generate the correction feedback signal that may correspond to the field-of-view frame, and wherein the correction feedback signal may include a suggested correction of the position of the field-of-view of the handheld intraoral scanner.

The dental object may relate to any object inside the oral cavity of a patient, such as a tooth, a part of a tooth, a plurality of teeth, an upper jaw or a lower jaw or parts thereof, gingiva or part of the gingiva, tongue, palate, a restoration and/or a preparation. The light information may relate to information received by the handheld intraoral scanner, during the scan session. The handheld intraoral scanner throughout the disclosure is also referred to as the scanner.

During the scan session the digital 3D model representing the dental object may be generated and updated by processing the light information received through the field-of-view of the handheld intraoral scanner. The digital 3D model may be displayed on the graphical user interface comprised in the intraoral scanner system. The field-of-view of the scanner may be understood as a maximum area the scanner can image.

By observing the graphical user interface the user of the intraoral scanner system, for example a dentist, may see, in real time, the displayed field-of-view frame corresponding to the position of the handheld intraoral scanner, more specifically its field-of-view, with respect to the oral cavity of the patient. Thereby, the position of the field-of-view frame with respect to the digital 3D model may correspond to the position of the field-of-view of the handheld intraoral scanner with respect to the dental object in the oral cavity of the patient. In this way the user has a reference, by viewing the graphical user interface, for where the scanner is with respect to dental objects in the oral situation.

During the scan session, the user may move the scanner away from the dental object which may be a scan target. As a consequence, generation of the digital 3D model may be interrupted, or the digital 3D model may be generated in a suboptimal manner from less scan data compared to obtainable scan data. In some cases, an incomplete digital 3D model may be generated with missing information.

Once the scanner moves in a manner such that the amount of collected scan data is reduced, the correction feedback signal may be generated. Interruption of the optimal generation (or buildup) of the digital 3D model may occur when the scanner is moved such that a part of the digital 3D model, rendered and thus visible through the field-of-view frame, is shifted away towards edges of the field-of-view frame. This may correspond to the fact that the scanner is moved such that the scan surface has reduced. The generated correction feedback signal may be associated with the displayed field-of-view frame and may serve as a guidance to the user on how to move the scanner to return to a position in the oral cavity from which the scanning can be resumed in an optimal manner, for example by exposing a larger surface area of teeth to the scanner and thus collecting more scan data. The correction feedback signal may be displayed such that it suggests correction of the position of the field-of-view of the handheld intraoral scanner. For example, the user operating the scanner may see, by observing the field-of-view frame, a moving trail of guiding elements such as rectangles, moving in a signal direction.

The correction feedback signal may be characterized by the signal direction and/or a signal intensity (these will be elaborated on later).

The signal direction may indicate a direction in which the user should move the scanner such as to resume acquisition of the scan data. The signal intensity may indicate by how much the user should move the scanner in the signal direction. The signal direction and/or the signal intensity may provide guidance to the user for performing effective scanning and optimal generation of the digital 3D model.

According to an embodiment, the intraoral scanner system may be configured to display the correction feedback signal during the scan session, wherein the intraoral scanner system may comprise:
- the handheld intraoral scanner configured to obtain light information reflected from the dental object through the field-of-view of the handheld intraoral scanner;
- the one or more processors configured to process the light information and to generate the digital 3D model of the dental object based on the processed light information; and
- the graphical user interface configured to display the digital 3D model,
wherein the one or more processors may be configured to:
- display in real-time and on the graphical user interface the field-of-view frame representing the position of the field-of-view of the handheld intraoral scanner; and
- generate the correction feedback signal if the handheld intraoral scanner deviates from a recommended scan path, wherein the correction feedback signal may be configured to guide the user to maneuver the handheld intraoral scanner to the recommended scan path.

The recommended scan path may be provided by the scanner manufacturer. The correction feedback signal may be configured to guide the user to maneuver the handheld intraoral scanner to a point on the recommended scan path where the deviation occurred.

According to an embodiment, the intraoral scanner system may be configured to display the correction feedback signal during the scan session, wherein the intraoral scanner system may comprise:
- the handheld intraoral scanner configured to obtain light information reflected from the dental object through the field-of-view of the handheld intraoral scanner;
- the one or more processors configured to process the light information and to generate the digital 3D model of the dental object based on the processed light information; and
- the graphical user interface configured to display the digital 3D model,

wherein the one or more processors may be configured to:
   - display in real-time and on the graphical user interface the field-of-view frame representing the position of the field-of-view of the handheld intraoral scanner; and
generate the correction feedback signal based on a relative position of a part of the digital 3D model rendered in the field-of-view frame with respect to the field-of-view frame.

The correction feedback signal may be configured to guide the user to maneuver the handheld intraoral scanner in a direction towards the position in the oral cavity from which further scanning should be continued so that the optimal build-up of the digital 3D model is resumed.

The signal direction and/or the signal intensity may be determined based on the relative position of the part of the digital 3D model rendered in the field-of-view frame with respect to the field-of-view frame. For example, the signal direction and/or the signal intensity may be determined based on the relative position of a center of mass of the part of the digital 3D model rendered in the field-of-view frame with respect to a center of the field-of-view frame.

The signal direction may be a direction from the center of the field-of-view frame towards the center of mass of the part of the digital 3D model rendered in the field-of-view frame.

The signal intensity may be a function of a distance between the center of the field-of-view frame and the center of mass of the part of the digital 3D model rendered in the field-of-view frame.

The correction feedback signal may comprise a shape and/or a dynamic pattern indicating the suggested correction of the position of the field-of-view of the handheld intraoral scanner.

The shape comprised in the correction feedback signal may be a geometrical shape such as a rectangle or a plurality of rectangles. Similarly, the shape may be an arrow. The dynamic pattern may refer to a moving pattern of shapes, for example rectangles or arrows, moving in the direction towards the position in the oral cavity from which further scanning should be continued so that the build-up of the digital 3D model is resumed. The shape and/or the dynamic pattern may comprise any geometrical shape.

In an embodiment the suggested correction of the position of the field-of-view of the handheld intraoral scanner may be displayed in form of the shape, for example a rectangle, and/or the dynamic pattern.

The suggested correction of the position of the field-of-view of the handheld intraoral scanner may include a direction or a rotation of the field-of-view of the handheld intraoral scanner.

The suggested correction of the position of the field-of-view of the handheld intraoral scanner may be an instruction to the user to maneuver the scanner towards the location in the oral cavity where more scan data can be captured, and the build-up of the digital 3D model on the graphical user interface can be resumed in the optimal way with the additional scan data. The instruction may comprise a translation and/or a rotation instruction for the movement of the handheld intraoral scanner. Thereby, the user may be instructed to maneuver the scanner in a certain direction according to the translation instruction and/or to rotate the scanner according to the rotation instruction. For example, the user may be prompted to move the scanner in an occlusal plane or any plane substantially parallel to the occlusal plane of the oral cavity. Additionally or alternatively, the user may be prompted to move the scanner closer or further away from the dental object, substantially along an axis which may be orthogonal to the occlusal plane.

In an embodiment the shape and/or the dynamic pattern may comprise the instruction comprising the translation instruction and/or the rotation instruction for the movement of the handheld intraoral scanner towards the position in the oral cavity from which further scanning should be continued so that the optimal build-up of the digital 3D model is resumed.

The one or more processors may be further configured to display the field-of-view frame. The field-of-view frame may be configured to display, in real-time, a region of the digital 3D model that is within the field-of-view of the handheld intraoral scanner. The user may thus be able to see, through the field-of-view frame, the part of the digital 3D model corresponding to the region of the oral cavity that is within the field-of-view of the handheld intraoral scanner.

The part of the displayed digital 3D model that is visible within the field-of-view frame may correspond to the region of the oral cavity that is within the field-of-view of the handheld intraoral scanner. Instead of the term "visible", term "rendered" may be used as the part of the digital 3D model may be rendered within the field-of-view frame. That part may be observable by the user.

In an embodiment the suggested correction may be determined by a trained neural network that is trained on a training data comprising optimal scan paths of intraoral scans and/or comprising optimal corrections for different scan situations, and wherein the trained neural network may be configured to determine the suggested correction based on the training data.

Thus, in an embodiment, the trained neural network may be configured to generate the suggested correction of the position of the field-of-view of the handheld intraoral scanner if the user moves the scanner away from the scan target. One role of the trained neural network may be to generate, based on the training data, improved scan paths. Improved scan paths may refer to predefined scan paths or dynamically generated scan paths during the scanning process.

In an example, the part of the digital 3D model rendered on the graphical user interface and visible through the field-of-view frame, may be segmented during the scanning process. In this way individual teeth and gingiva may be recognized as separate three-dimensional objects. The trained neural network may predict, based on the segmented objects in the field-of-view frame, a specific position in the three-dimensional space lying on the scan path, towards which the user should move the scanner. The trained neural network may, by having information about the tooth in the field-of-view frame, predict where a neighboring tooth should be located, and output the suggested correction directed to the prediction. The information about the tooth may be information about type of the tooth (for example incisor, molar, pre-molar, canine), information about location of a centroid of the tooth and/or information about orientation of the tooth in the jaw.

In an example, the suggested correction may be based on a prediction of a location of a neighboring dental object to the dental object being scanned.

In an example the part of the digital 3D model rendered on the graphical user interface and visible through the field-of-view frame may be segmented to identify a tooth. Furthermore, a centroid of the tooth may be identified. Next, a location of a neighboring tooth may be predicted based on the identified tooth. Specifically, the location of the centroid of the neighboring tooth may be predicted. The feedback correction signal may be generated, directed from the centroid of the tooth towards the centroid of the neighboring tooth.

Information such as recommended scan paths, for example provided by scanner manufacturers, may be used as the training data for designing the trained neural network configured to provide the suggested correction. Information about surface geometry of variety of teeth (variety of 3D scans) may comprise part of the training data. Optimal corrections for different scan situations may be derived from the training data.

The suggested correction may be based on a scan path. The scan path may define a path for the field-of-view of the handheld intraoral scanner along the dentition in the oral cavity.

The scan path may be one of:
- a default scan path that is predefined by the manufacturer or the user of the system;
- a custom scan path that is determined for an individual scan session;
- a selectable scan path that is selected amongst several selectable scan paths for different dentitions; or
- a dynamic scan path that is configured to be modified during a scan session by the one or more processors to adapt relative to a current position of the field-of-view of the handheld intraoral scanner.

In an embodiment, the suggested correction may be directed towards the scan path for the field-of-view of the handheld intraoral scanner, wherein the scan path is one of:
- the default scan path that is predefined by the manufacturer or the user of the system;
- the custom scan path that is determined for the individual scan session;
- the selectable scan path that is selected amongst several selectable scan paths for different dentitions; or
- the dynamic scan path configured to be modified during the scan session by the one or more processors to adapt relative to the current position of the field-of-view of the handheld intraoral scanner.

The custom scan path may be a scan path based upon at least one earlier scan of a same patient. The custom scan path may be based on needs and/or desires of the user (e.g. dentist).

The selectable scan path may be selected based on specific needs of a patient. The selectable scan path may, for example, be selected for edentulous patients. Scanning edentulous patients may require a specific scan strategy in order to achieve a desired scan accuracy, for example including a slow and steady scanning of the palate. Depending on what purpose the scanning process serves, a selectable scan path may be provided. For example, the selectable scan path may be provided for preparation scanning. In scanning of the preparation it may be crucial to acquire detailed information about a preparation line so an accurate digital 3D model of the preparation and a restoration can be generated.

The dynamic scan path may be a scan path that changes and adapts during the scanning process. A part of the digital 3D model needing additional scanning may be determined and the suggested correction may be generated towards the determined part of the digital 3D model. In a case where the generation of the digital 3D model is interrupted, for example due to lack of scan data from a specific location needed for continued rendering of the digital 3D model, the correction feedback signal may be generated with the signal direction towards the specific location. In this way the user may, by following the correction feedback signal, move the scanner to the specific location such that the lack of scan data may be overcome.

The one or more processors may be configured to determine the deviation of the position of the field-of-view of the handheld intraoral scanner from the scan path. The one or more processors may further be configured to determine the suggested correction of the position of the field-of-view of the handheld intraoral scanner based on the determined deviation and the current position of the field-of-view of the handheld intraoral scanner. The current position of the field-of-view of the handheld scanner may correspond to the position of the field-of-view frame with respect to the part of the digital 3D model.

**In** an example, the one or more processors may be configured to determine the suggested correction of the position of the field-of-view of the handheld intraoral scanner based on the determined deviation.

**In** an example, the deviation of the position of the field-of-view of the handheld intraoral scanner from the scan path may be understood as the shortest distance from the position of the field-of-view of the handheld intraoral scanner from the scan path.

The user, while holding the scanner, may deviate from the scan path. Depending on the level of deviation, the suggested correction may be generated such that the user is able to return to the scan path.

The one or more processors may be configured to determine the custom scan path for the individual scan session by receiving individual dentition information that may include surface information of the dental object and arrangement of the dentition. Further, the one or more processors may be configured to determine, based on the individual dentition information, the custom scan path. The custom scan path may be advantageous if the user has a tendency to deviate from the recommended (default) scan path in a particular manner. This behavior may be detected, for example by the trained neural network, and the custom scan path may be generated to correct for the undesirable behavior.

The one or more processors may be further configured to determine a region in the field-of-view frame that may comprise the digital 3D model of the dental object or a part thereof, determine a direction and/or orientation of the field-of-view of the handheld intraoral scanner for centralizing the region within the field-of-view frame, and generate the suggested correction based on the determined direction and/or orientation of the field-of-view of the handheld intraoral scanner. In general, it may be desirable to centralize the part of the digital 3D model observable through the field-of-view in the center of the field-of-view frame. The generated correction feedback signal may be configured to indicate, on the graphical user interface, movement of the scanner needed in order to centralize the part of the digital 3D model that is observable through the field-of-view frame, around the center of the field-of-view frame. In effect, larger surface area is exposed to the field-of-view of the scanner and more scan data is captured such that the digital 3D model can be built in the optimal way.

The one or more processors may further be configured to determine an area of the digital 3D model comprising missing information, determine the direction and/or orientation of the field-of-view of the handheld intraoral scanner towards the area of the digital 3D model, and generate the suggested correction based on the determined direction and/or orientation. The area of the digital 3D model comprising missing information may be a part of the digital 3D model comprising empty spaces (holes) due to lack of scan data needed to generate the complete digital 3D model.

Generating the digital 3D model comprising the area with missing information may be a result of incomplete light information capture by the scanner. Thus, the insufficient amount of light information received by the scanner may result in generation of the digital 3D model that is incomplete, for example comprising empty regions also called "holes". The suggested correction in this case may be reflected in the field-of-view frame arranged in the direction towards the area of the digital 3D model with missing information, such that the user may react according to the suggested correction and move the scanner to the corresponding position in the oral cavity.

The one or more processors may be configured to determine a first position of a reference dental object of a reference digital 3D model, and wherein the reference digital 3D model may include expected positions of reference dental objects, determine a second position on the generated digital 3D model that may correspond to the first position on the reference digital 3D model, and wherein the generated digital 3D model may not comprise a dental object at the second position as may be indicated by the first position, and generate the suggested correction based on the second position on the generated digital 3D model. The reference digital 3D model may be a previously generated digital 3D model of the same patient. The reference digital 3D model may be segmented into individual reference dental objects such as reference teeth and gingiva. During the scanning process, the one or more processors may use information about the reference dental object and/or the reference digital 3D model, to generate the suggested correction of the correction feedback signal.

The one or more processors may utilize a trained neural network configured to determine the first position and the second position. The trained neural network may be trained based on a plurality of digital 3D models of different dentitions. The plurality of digital 3D models may include different arrangements and surfaces of teeth of the different dentitions.

The indication of the suggested correction of the position of the field-of-view of the handheld intraoral scanner may comprise one or more of a geometric object, an arrow, or a lightning effect. In this manner, the user may be guided towards the optimal scan path by following the suggested correction.

The dynamic pattern may comprise moving objects that may be configured to move along the signal direction.

In an example, the dynamic pattern may comprise moving objects configured to move towards or from the field-of-view frame in the signal direction. In this way, the user may be assisted to maneuver the scanner towards the position where scanning may have been interrupted.

The moving objects may be configured to increase in size during the movement towards or from the field-of-view frame if a distance between a scan tip of the handheld intraoral scanner and the dental object being scanned exceeds a predetermined distance limit.

In an example, the dynamic pattern may include multiple shapes for example rectangles or arrows. A distance between each of the shapes may increase when a distance between the position of the field-of-view of the handheld intraoral scanner and the suggested position increases. The distance between each of the shapes may decrease when the distance between the position of the field-of-view of the handheld intraoral scanner and the suggested position decreases.

The distance between each of the shapes in the correction feedback signal may also be referred to as the signal intensity.

The one or more processors may be configured to generate a second field-of-view frame within the field-of-view frame if a distance between a scan tip of the handheld intraoral scanner and the dental object being scanned exceeds a distance limit which may be predetermined. The predetermined distance limit in this case means a distance limit substantially along the axis orthogonal to the occlusal plane. The distance in this case may be referred to as a height distance. The predetermined distance limit may, in an example, be a specific depth-of-field of the scanner. In an example the depth-of-field of the scanner may be in the range of 10-20 millimeters, preferably in the range of 15-18 millimeters. Depending on the technology employed in the scanner, depth-of-field may be larger than 18 millimeters. Generally, the predetermined distance limit may relate to a distance between the scanner and the dental object (scan target) at which maximum amount of scan data may be collected from the dental object.

A size of the second field-of-view frame may change as the height distance changes relative to the predetermined distance limit.

The one or more processors may be configured to increase a frequency of the dynamic pattern when the field-of-view of the handheld intraoral scanner moves closer to the suggested position. The frequency may refer to frequency of movement of the shapes within the dynamic pattern.

The one or more processors may be configured to change the shape of the correction feedback signal to comprise a curved object when the suggested correction comprises a rotation of the field-of-view of the handheld intraoral scanner.

The one or more processors may be configured to change the dynamic pattern of the correction feedback signal to comprise an object moving along a curved path when the suggested correction comprises the rotation of the field-of-view of the handheld intraoral scanner.

The one or more processors may be configured to generate a haptic and/or a visual signal in the handheld intraoral scanner that may correspond to the correction feedback signal when generated, and/or an audio signal in the handheld intraoral scanner and/or a device displaying the graphical user interface that may correspond to the correction feedback signal when generated.

The one or more processors may be configured to perform a registration of the obtained light information for generating the digital 3D model. The registration may correspond to a registered region of the digital 3D model. The one or more processors may perform a partial registration of the obtained light information for generating the digital 3D model. The partial registration may correspond to a partly registered region of the digital 3D model. The one or more processors may be configured to detect a loss of registration of the obtained light information. The one or more processors may be configured to generate the suggested correction including a position of the partly registered region when the loss of registration is detected.

The one or more processors may generate an incomplete digital 3D model due to missing scan data from a part of the oral cavity. This may happen for example if the user misses scanning the part of the oral cavity and no light information or incomplete light information is received from that part of the oral cavity. The generated incomplete digital 3D model may comprise an empty space (a hole) corresponding to the part of the oral cavity that has not been scanned or has been partially scanned. The one or more processors may detect loss of light information and may generate, based on the detected loss, the correction feedback signal. The correction feedback signal may be configured to dynamically guide the user towards the part of the oral cavity from which no light information, or insufficient amount of light information, is received. The correction feedback signal may be displayed in the graphical user interface.

The one or more processors may be configured to display the field-of-view frame comprising a shape that mimics a shape of the field-of-view of the handheld intraoral scanner.

The one or more processors may be configured to display the field-of-view frame comprising a shape that mimics a shape of a window of the handheld intraoral scanner, wherein the window may be configured to facilitate a passing of the light information reflected from the dental object.

In an embodiment, a method for generating the correction feedback signal during the scan session is disclosed, wherein the method comprises:
- obtaining the light information reflected from the dental object inside the oral cavity through the field-of-view of the handheld intraoral scanner,
- processing the light information and generating the digital 3D model of the dental object based on the processed light information,

- displaying in real-time and on the graphical user interface the field-of-view frame representing the position of the field-of-view of the handheld intraoral scanner,
- generating the correction feedback signal that corresponds to the field-of-view frame, wherein the correction feedback signal includes the suggested correction of the position of the field-of-view of the handheld intraoral scanner.

The correction feedback signal may be characterized by a signal direction, wherein the signal direction may be a direction from a center of the field-of-view frame towards a center of mass of a part of the digital 3D model rendered in the field-of-view frame or a direction parallel to the direction from the center of the field-of-view frame towards the center of mass of the part of the digital 3D model rendered in the field-of-view frame.

Generating the correction feedback signal may comprise displaying the correction feedback signal on the graphical user interface, for example associated with the field-of-view frame. The correction feedback signal may be displayed with the signal direction and/or the signal intensity. The correction feedback signal may thus provide a clear guidance to the user on how to move the scanner for an effective, fast scanning process and resulting generation of an accurate digital 3D model.

A non-transitory computer-readable medium is disclosed, comprising instructions which, when executed by a computer, cause the computer to carry out the method according to any one or more of the disclosed embodiments.

A computer program product is disclosed, comprising instructions which, when the program is executed by a computer, causes the computer to carry out the method of any one or more of the disclosed embodiments.

### BRIEF DESCRIPTION OF THE DRAWINGS

FIG. 1 schematically illustrates scanning of an intraoral cavity with a handheld intraoral scanner and a corresponding digital 3D model displayed on a graphical user interface;
FIG. 2 illustrates a scan path;
FIG. 3A, 3B illustrate a correction feedback signal with a signal direction and a signal intensity;
FIG. 3C, 3D illustrate the digital 3D model, a field-of-view frame representing a position of a field-of-view of the handheld intraoral scanner and an example of the correction feedback signal;
FIG. 4A, 4B illustrate the digital 3D model, the field-of-view frame representing the position of the field-of-view of the handheld intraoral scanner and a further example of the correction feedback signal;
FIG. 5A, 5B illustrate the digital 3D model, the field-of-view frame representing the position of the field-of-view of the handheld intraoral scanner and a further example of the correction feedback signal;
FIG. 6 illustrates a method for generating the correction feedback signal during the scan session;
FIG.7 illustrates an intraoral scanner system.

### DETAILED DESCRIPTION OF THE DRAWINGS

In the following description, reference is made to the accompanying figures, which show by way of illustration how the invention may be practiced.

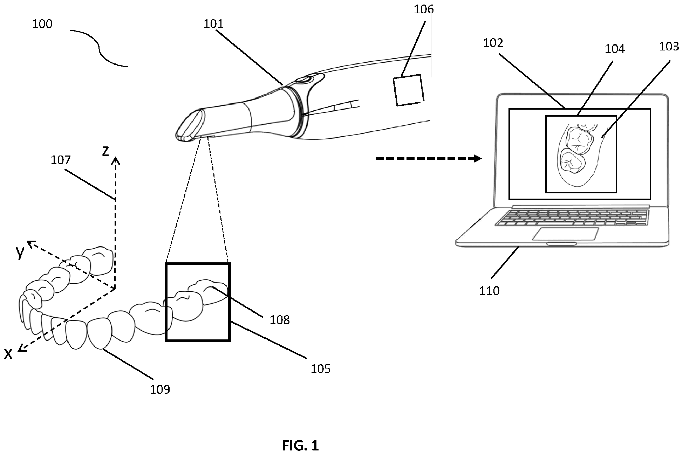

Figure 1 illustrates a part of the intraoral scanner system 100, namely a handheld intraoral scanner 101 and a graphical user interface 102 which may be a part of a computer device, for example a laptop computer 110. The handheld intraoral scanner 101, also referred to as a scanner, may be configured for scanning an oral cavity of a patient. As illustrated in figure 1, teeth of lower jaw 109 of the patient's oral cavity may be scanned.

The scanner 101 may be configured to obtain light information reflected from a dental object 108 inside the oral cavity through a field-of-view 105 of the scanner 101. The dental object 108 may be any tooth or a part thereof, a plurality of teeth, gingiva, a preparation, a palate, a restoration or any other object in the oral cavity. The intraoral scanner system 100 may comprise one or more processors 106 configured to process the light information and to generate a digital 3D model 103 of the dental object 108 based on the processed light information. The one or more processors 106 may be in the scanner 101 (as illustrated in figure 1), the computer device displaying the graphical user interface 102 and/or may be a part of a remote computer resource.

A region (surface) of the oral cavity instantaneously captured by the scanner 101 may be defined by the field-of-view 105 of the scanner 101, shown as rectangle in figure 1. The scanner 101 may receive the light information from the dental object 108 within the field-of-view 105 of the scanner 101. Based on the received light information, the one or more processors 106 may generate the digital 3D model 103. The generated digital 3D model 103 may represent the surface of the dental object 108. As a user, for example a dentist, moves the scanner 101 within the oral cavity, scanner's field-of-view may cover remaining regions of the oral cavity and the digital 3D model 103 may be updated accordingly.

The scanner 101 may be based on any known scanning principle such as focus scanning, triangulation, confocal scanning, stereo vision, structure from motion.

For example, the scanner 101 may be based on a focus scanning principle where the scanner 101 may comprise an optical element, such as a focus lens, configured to move back and forth during scanning to change the focus of the scanner 101, whereby depth information can be estimated based on a focus measure. A focus scanner is further described in EP 2 442 720 B1. In some examples, the scanner 101 may be based on a depth-from-defocus scanning principle, wherein an optical property, such as an aperture, is changed between the acquisition of two images, whereby depth can be estimated by determining the degree of defocus between the two images. A focus scanner may be characterized by a relatively narrow depth-of-field property which may define a volume in which the dental object may be, for it to be in-focus while being scanned. The depth-of-field of the focus scanner may be understood as a depth interval from 0 to a maximum depth-of-field. In an example the interval may be 0 to 20 millimeters, where 20 millimeters is maximum depth-of-field.

In some examples, the scanner 101 may be based on triangulation, wherein at least one camera and a projector unit are positioned such that they form a triangle with respect to a point on the scanned surface. As an example, a projector and a camera may be utilized to determine points in 3D space based on triangulation. Alternatively, the scanner 101 may comprise two or more cameras viewing the scene or scanned object from two different directions, wherein the cameras are configured to acquire a set of images, wherein a correspondence problem is solved based on triangulation. The correspondence problem generally refers to the problem of ascertaining which parts of one image correspond to which parts of another image. Specifically, the projector unit may be configured to project a plurality of projector rays, which are projected onto a surface of the dental object. In particular, solving the correspondence problem may include the steps of determining image features in the images within a set of images, and further associate said image features with a specific projector ray. Subsequently, the depth of each projector ray may be computed, whereby a 3D representation of the scanned object may be generated. A triangulation scanner may be characterized by a deeper depth-of-field property compared to a focus scanner.

The scanner 101 may generate a plurality of two-dimensional (2D) images of the dental object 108 which may be subsequently joined together to create the digital 3D model 103. This process of combining the 2D images together may be referred to as "stitching" or registration. Registering of the 2D images may comprise joining together the 2D images having overlapping portions.

The movement of the scanner 101 within the oral cavity may comprise a complex set of motions in the three-dimensional space. Some simplified examples of possible scanner 101 movements are mentioned next. The movement of the scanner 101 within the oral cavity may be observed through a coordinate system defined by three orthogonal axes x, y and z, shown in figure 1 as set for example in the lower jaw 109. The movement may comprise translational movement along the mentioned axes and/or rotational movement around the mentioned axes. The scanner 101 may be moved in a plane substantially parallel to a XY plane defined by axes x and y, to scan occlusal and incisal surfaces of teeth, also referred to as biting surfaces of the teeth. The scanner 101 may be moved substantially along the z axis closer to, or further away from the scan target, for example from the dental object 108 in figure 1. To scan facial (buccal) or lingual surfaces, the scanner may be rotated such that those surfaces are within scanner's field-of-view 105.

The one or more processors 106 may be configured to display, on the graphical user interface 102, a field-of-view frame 104 that may represent a position of the field-of-view 105 of the scanner 101. In this way, the user may know exactly what region of the oral cavity is within the scanner's field-of-view 105 by observing the field-of-view frame 104. Thus, movement of the scanner 101 may result in the corresponding movement of the field-of-view frame 104 on the graphical user interface 102.

The field-of-view frame 104 may be displayed overlaying the digital 3D model 103 being rendered, as shown in figure 1. Additionally or alternatively, the field-of-view frame 104 may be displayed separately from the digital 3D model 103 being rendered on the graphical user interface 102. Such separately displayed field-of-view frame 104 may indicate the position of the field-of-view 105 of the scanner 101 by showing a 2D image or a plurality of 2D images or a stream of 2D images as they are captured by the scanner 101. In general, the field-of-view frame 104 may be displayed on the graphical user interface 102 and may indicate the position of the scanner with respect to the dental object 108 being scanned.

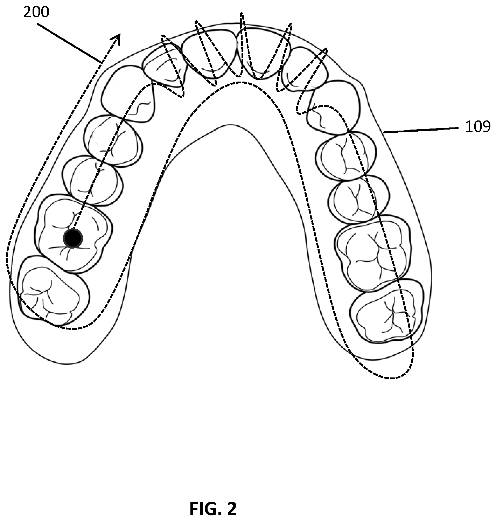

Figure 2 illustrates an example of a scan path, in this case for the patient's lower jaw 109. The scan path 200 illustrated in figure 2 may be referred to as a recommended scan path and may be suggested for use by scanner manufacturers in order to mitigate inherent accuracy deficiencies of the scanner 101 and to help users learn best practice for fast and accurate scanning. The scan path 200 comprises a curved part over front (incisor) teeth due to a particular challenge to scan incisor teeth as they are characterized by small incisal surface area. The user may move the scanner 101 according to the scan path 200 to complete scanning of the lower jaw 109. The recommended scan path for an upper jaw may be similar or same as the scan path 200 for the lower jaw 109.

An experienced user may operate the scanner 101 by following the scan path 200 such that all relevant information about surface geometry of the oral cavity is captured and the complete digital 3D model 103 may, in turn, be generated. However, in certain situations, generation of the digital 3D model 103 may be based on insufficient scan data, or even interrupted, due to variety of reasons. For example, an inexperienced user may move the scanner 101 completely away from teeth, such that the teeth are completely outside of the scanner's field-of-view 105. This may be visible as a loss of information in the field-of-view frame 104 on the graphical user interface 102. For example, no data may be visible through the field-of-view frame 104. Consequently, the generation of the digital 3D model 103 may be interrupted.

When the user starts moving the scanner 101 away from the teeth, less data is being collected, which may result in the generation of the digital 3D model 103 with sub-optimal amount of scan data. It may be desirable for the user to obtain guidance on how to move the scanner 101 back towards a location in the oral cavity so that larger teeth surface is scanned and the generation of the digital 3D model 103 can be resumed based on more captured data. This guidance may be desirable as soon as the user starts moving the scanner 101 away from the teeth. A solution in the form of a correction feedback signal is offered by an embodiment of the disclosure.

The location in the oral cavity towards which the scanner 101 may be directed may be the one with larger surface area of the dental object 108 to be scanned, compared to the current position of the scanner 101.

It may occur that the user moves the scanner 101 away from the teeth in such amount that generation of the digital 3D model 103 is interrupted. In that case, it may be desirable for the user to obtain guidance on how to move the scanner 101 back towards the location in the oral cavity so that generation of the digital 3D model 103 can be continued. As a solution, the correction feedback signal may be generated, based on a last known position of the scanner 101, to guide the user to move the scanner 101 resulting in resuming generation of the digital 3D model 103.

The user may also hold the scanner 101 at a distance too far away, substantially along the z axis 107, from the dental object 108, that may be scan target. In that case, it may be desirable for the user to receive guidance on how to move the scanner 101 closer to the dental object 108 along the z axis 107. This may be particularly useful for scanners with narrow depth-of-field where slight movements of the scanner 101 along the z axis 107 may result in the dental object 108 appearing out of focus. This may happen, for example, if the distance from the scanner tip to the dental object 108 is greater than the depth-of-field of the scanner 101. Such instruction may be particularly advantageous when scanning uneven surfaces or when the scanner 101 is angled to capture buccal and/or lingual surfaces of teeth. In some cases, it may be desirable for the user to receive an instruction to move the scanner 101 further away from the dental object 108 such that more data can be collected.

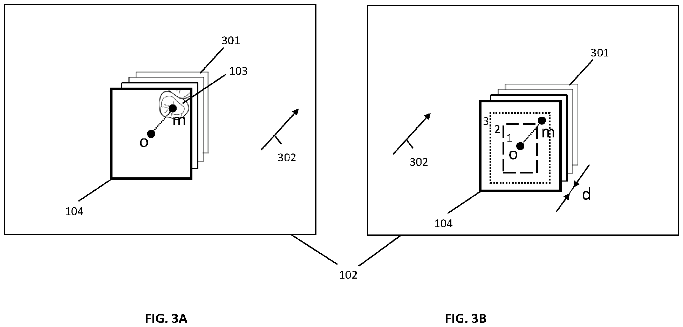

The one or more processors 106 may be configured to generate the correction feedback signal 301 (shown for example in figure 3A, 3B) that may correspond to the field-of-view frame 104. The correction feedback signal 301 may include a suggested correction of the position of the field-of-view 105 of the scanner 101. The correction feedback signal 301 may thus be displayed in a relation to the field-of-view frame 104, such as over the field-of-view frame 104, at one or more points on a border of the field-of-view frame 104, at one or more points inside the field-of-view frame 104 for example in a center of the field-of-view frame 104.

In an example, the correction feedback signal 301 may be associated with the field-of-view frame 104 and may appear depending on the position of the scanner 101 with respect to the dental object 108 which may be the scan target.

The position of the scanner 101 with respect to the dental object 108 may be observed by viewing the field-of-view frame 104 on the graphical user interface 102.

The correction feedback signal 301 may be characterized by a signal direction 302 and a signal intensity. The correction feedback signal may appear as a visually recognizable signal associated to the field-of-view frame 104 on the graphical user interface 102. For example, the correction feedback signal 301 may be in form of a plurality of rectangles attached to the field-of-view frame 104. The signal direction 302 may determine orientation of the correction feedback signal 301. The signal intensity may indicate different level of movement of the scanner 101, required from the user in order to return the scanner 101 to the position in the oral cavity from which generation of the digital 3D model 103 can be continued, for example with increased amount of scan data.

The correction feedback signal 301 may be a dynamic signal. For example, its shape may be moving along the signal direction 302.

Figure 3A illustrates the correction feedback signal 301 in form of a plurality of rectangles attached to the field-of-view frame 104. The correction feedback signal is oriented in the signal direction 302.

In an example, the signal direction 302 may be determined from the relative position of a part of the digital 3D model 103 visible in the field-of-view frame 104, and the field-of-view frame 104 itself. In the example illustrated in figure 3A, the signal direction 302 is determined by a line connecting a center (o) of the field-of-view frame 104 and a center of mass (m) of the part of the digital 3D model 103 rendered in the field-of-view frame 104.

Therefore, in order to determine the signal direction 302, first the center of mass (m) of the part of the digital 3D model 103 rendered in the field-of-view frame 104 may be determined. Then, the signal direction 302 may be determined by connecting the center (o) of the field-of-view frame 104 and the center of mass (m) of the part of the digital 3D model 103 rendered in the field-of-view frame 104. The signal direction 302 may then be a direction from the center (o) to the center of mass (m).

The part of the digital 3D model 103 rendered in the field-of-view frame 104 may refer to the part of the digital 3D model that is in focus in the field-of-view frame 104.

Figure 3B illustrates an example of determining the signal intensity of the correction feedback signal 301. The signal intensity may be understood as a distance (d) between each of the plurality of shapes, for example rectangles comprised in the correction feedback signal 301. In one example, the signal intensity may depend on a location of the center of mass (m) of the part of the digital 3D model 103 rendered in the field-of-view frame 104 (the part of the digital 3D model 103 not shown in figure 3B but only its center of mass m). The surface area of the field-of-view frame 104 may be divided into a number of zones which may be of same shape as the field-of-view frame 104 and may have the same center (o) as the field-of-view frame 104 (concentric zones). The signal intensity may then have a first value if the center of mass (m) is in zone 1. The first value may be zero and the correction feedback signal 301 may not appear in such case. If the center of mass (m) lies in zone 2 then the signal intensity may have a second value, higher than the first value. If the center of mass (m) lies in zone 3 then the signal intensity may have a third value, higher than the second value. There may be defined a different number of zones than the three zones shown in figure 3B.

In another example, the signal intensity may be a function of a distance (o, m) between the center (o) of the field-of-view frame 104 and the center of mass (m) of the part of the digital 3D model 103 rendered in the field-of-view frame 104:$signal intensity = distance \left(o, m\right) \times \frac{maximum intensity}{maximum distance}$ wherein the maximum intensity is a maximum value of the signal intensity. The maximum intensity may be a value for the maximum distance (d) between each of the plurality of rectangles comprised in the correction feedback signal 301, expressed in millimeters. It may be pre-defined or set by the user. The correction feedback signal 301 may have the maximum intensity when the distance between the center (o) of the field-of-view frame 104 and the center of mass (m) of the part of the digital 3D model 103 rendered in the field-of-view frame 104 is maximum. That may occur when the center of mass (m) of the part of the digital 3D model 103 rendered in the field-of-view frame 104a lies on a furthest point from the center (o), on the field-of-view frame 104.

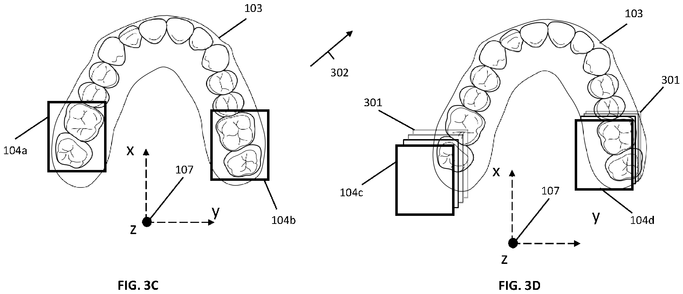

Figure 3C illustrates the digital 3D model 103, the field-of-view frame 104a in a first position and the field-of-view frame 104b in a second position. These two positions may represent two correct positions of the field-of-view 105 of the scanner 101 lying on the scan path 200. The parts of the digital 3D model 103, visible through the fields-of-view frame 104a, 104b are centralized within their respective fields-of-view. In these cases, no correction feedback signal 301 is generated.

Figure 3D illustrates the digital 3D model 103, the field-of-view frame 104c in a third position and the field-of-view frame 104d in a fourth position. These two positions may represent two non-optimal positions of the field-of-view 105 of the scanner 101, for example with respect to the scan path 200 because the scanner 101 collects less data compared to positions indicated by the scan path 200. It may be visible that parts of the digital 3D model 103 visible through the field-of-view frame 104c and the field-of-view frame 104d are not centralized around the center points of their respective field-of-view frames. In both positions of the field-of-view frame 104c, 104d, the correction feedback signal 301 may be generated. The correction feedback signal 301 may be directed along the signal direction 302 which can be determined as previously described. The correction feedback signal may indicate a direction in which the scanner 101 should be moved, within the XY plane, for the field-of-view 105 of the scanner 101 to return to the position in the oral cavity from which further scanning should be continued so that optimized build-up of the digital 3D model 103 is resumed.

In an embodiment, as seen in figures 3A, 3B, 3D the correction feedback signal 301 may comprise a geometrical shape, for example a rectangle. Plurality of shapes, for example rectangles, may be arranged in a pattern to comprise the correction feedback signal 301. The shapes in the pattern may be of varying opacity. Furthermore, the shapes in the pattern may be moving to or from the field-of-view frame 104, thus forming a dynamic pattern. The shapes in the pattern may be arranged along the signal direction 302 which may be associated with suggested correction of the position of the field-of-view 105 of the scanner 101. Thus the shapes may be generated if the field-of-view 105 of the scanner 101 moves such that the part of the digital 3D model visible through the field-of-view frame 104 gets shifted away from the center of the field-of-view frame 104. Moving of the scanner 101 in this case may be in the plane substantially parallel to the XY plane defined by axes x and y.

A distance between the shapes, for example rectangles, in the correction feedback signal 301 may be understood as the signal intensity of the correction feedback signal 301. Depending on the position of the part of the digital 3D model 103, visible through the field-of-view frame 104 relative to the field-of-view frame 104, the signal intensity may be higher or lower. As can be seen in figure 3D, the field-of-view frame 104c indicates a position of the scanner 101, more precisely of a scanner tip, that deviates from the optimal position where the scanner tip should be, for example to collect data from a larger scan surface. The correct position of the scanner tip, for example lying on the scan path 200, may be indicated with the field-of-view frame 104a in figure 3A. Deviation in this case may be understood as a deviation in the plane substantially parallel to the XY plane. Arrangement of the shapes in the correction feedback signal 301 associated with the field-of-view frame 104c, and their movement in the signal direction 302 may indicate to the user that the scanner 101 should be moved substantially along a direction corresponding to the signal direction 302.

To be additionally seen in figure 3D is the field-of-view frame 104d indicating a position of the scanner 101, more precisely of the scanner tip, that deviates from the optimal position where the scanner tip should be. The optimal position of the scanner tip may be a position corresponding to the field-of-view frame 104b in figure 3C. Deviation in this case may be understood as a deviation of the scanner tip in the plane substantially parallel to the XY plane. Arrangement of the shapes in the correction feedback signal 301 associated with the field-of-view frame 104d, and their movement in the signal direction 302 may indicate to the user that the scanner 101 should be moved substantially along the direction corresponding to the signal direction 302. Distance between the shapes, in this case rectangles, in the correction feedback signal 301 associated with the field-of-view frame 104d is less than the distance between the shapes, shown as rectangles, in the correction feedback signal 301 associated with the field-of-view frame 104c. Thus, the deviation of the scanner tip position indicated by the position of the field-of-view frame 104c, from the optimal position indicated by the position of the field-of-view frame 104a, is larger than the deviation of the scanner tip position indicated by the position of the field-of-view frame 104d, from the correct position indicated by the position of the field-of-view frame 104b. The user may therefore react, observing the correction feedback signal 301, with a movement across a greater distance when holding the scanner 101 in the position corresponding to the field-of-view frame 104c than in the position corresponding to the field-of-view frame 104d. The user may be prompted, in both cases, to move the scanner 101 in the plane parallel to the XY plane in the direction corresponding to the signal direction 302.

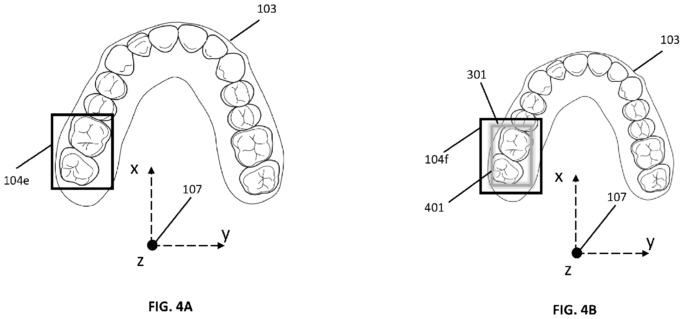

Figure 4A, similar to figure 3A, illustrates the digital 3D model 103 and the field-of-view frame 104e associated with the optimal positions of the field-of-view 105 of the scanner 101. The part of the digital 3D model 103, visible through the field-of-view frame 104e is centralized around the center of the field-of-view frame 104e. This position of the scanner 101may also lie on the scan path 200. In this case, no correction feedback signal 301 is generated.

Figure 4B illustrates the digital 3D model 103 and the field-of-view frame 104f associated with the position of the field-of-view 105 of the scanner 101 which may be the optimal position with respect to the scan path 200 in the plane substantially parallel to the XY plane. However, this position of the scanner 101 may be non-optimal along the z axis 107, for example because less scan data is collected by moving the scanner 101 further away from the dental object 108, along the z axis 107. Thus, the scanner 101 is positioned such that an optimal scan height along the z axis 107 is not reached. This situation may be indicated to the user via the correction feedback signal 301 that may comprise a scan frame gap between the field-of-view frame 104f and a second frame 401 which may be concentric to the field-of-view frame 104f.

The second frame 401 may also be referred to as the second field-of-view frame 401 throughout the disclosure.

The second frame 401 may be generated if the scanner 101, or more precisely the scanner tip, is at a distance from the scan target such that the digital 3D model 103 is generated with less scan data than optimal. The shape of the second frame 401 may correspond to that of the field-of-view frame 104f. The second frame 401 may be scaled with respect to the field-of-view frame 104f to indicate the suggested correction of the position of the field-of-view 105 of the scanner 101 along the z axis 107. For example, as indicated in figure 4B, the second frame 401 is scaled down, together with the digital 3D model 103, to indicate that the scanner height is not optimal. In this case the scanner 101 may be held too high with respect to the dental object 108. The scan frame gap that may appear may thus indicate the distance between the height at which the scanner 101 is positioned and the position where the scanner 101 should be held such that the optimal scan height is reached. This provides clear guidance to the user to adjust the scan height.

The correction feedback signal 301 may thus comprise the scan frame gap to indicate the correction of the position of the field-of-view 105 of the scanner 101 such that, when the correction is applied by the user, the optimal scan height is reached. It may be understood that the correction feedback signal 301 may comprise, in addition to the scan frame gap, also the plurality of shapes arranged along the signal direction 302. This component of the correction feedback signal 301 may appear to suggest movement of the scanner 101 in the plane substantially parallel to the XY plane.

The correction feedback signal 301 may comprises the scan frame gap between the field-of-view frame 104f and the second frame 401 to indicate the correction of the position of the field-of-view 105 of the handheld intraoral scanner 101 such that, when the correction is applied by the user, the distance between the scan tip of the handheld intraoral scanner 101 and the dental 108 object being scanned is less than the predetermined distance limit.

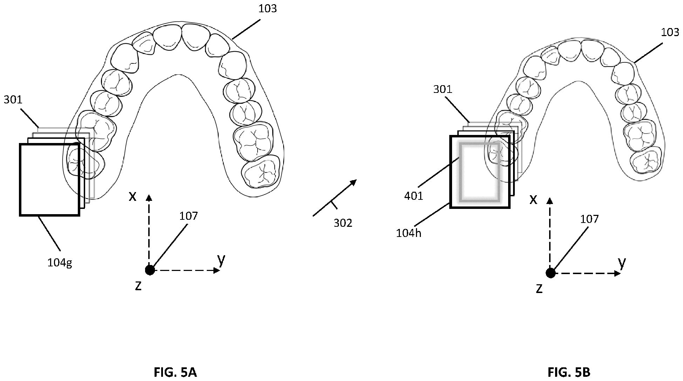

Figure 5A illustrates the correction feedback signal 301 associated with the field-of-view frame 104g, comprising the plurality of shapes, in this case rectangles, arranged along the signal direction 302, thereby indicating a direction in which the user should move the scanner 101 to return to the position in the oral cavity from which the further build-up of the digital 3D model 103 can be continued in an optimal way. The rectangles may be of varying opacity and/or may be arranged to move towards or away from the field-of-view frame 104g. In the case of figure 5A, the correction feedback signal 301 may indicate to the user that the scanner 101 should be moved in the plane substantially parallel to the XY plane in the direction of the shapes coinciding with the signal direction 302. No adjustment of the scan height is required as the correction feedback signal 301 does not comprise the frame gap.

It may be understood that the correction feedback signal 301 may comprise any geometrical shape or plurality of shapes, such as lines, arrows, triangles, circles etc.

Contrary to figure 5A, figure 5B illustrates the correction feedback signal 301 indicating to the user that, besides the correction in the plane substantially parallel to the XY plane, a further correction of the scan height may be required. Correction of the scan height may be indicated with the frame gap appearing between the field-of-view frame 104h and the second frame 401. The second frame 401 may be scaled down with respect to the field-of-view frame 104h. Optionally, the digital 3D model 103 may be scaled down proportionally to the second frame 401. In the case of figure 5B, the correction feedback signal 301 may comprise plurality of shapes, for example rectangles, that decrease in size along the signal direction 302. This may be an indication to the user that both types of movements are required for position correction. By responding to the correction feedback signal 301 of figure 5B the user may move the scanner 101 in the plane substantially parallel to the XY plane, in the direction indicated by the plurality of rectangles to bring the scanner 101 to the correct position such that the part of the digital 3D model 103 is centralized around the center of the field-of-view frame 104h. Additionally, the user may, observing the displayed scan gap, lower the scan height along the z axis 107, for example by bringing the scanner 101 closer to the dental object 108 such that scan gap disappears. By moving the scanner 101 closer to the dental object 108 (scan target) maximal amount of scan data from the scan target can be captured. The user may in this way be presented with a suggestion for moving the scanner 101 to the correct position where the optimal build-up of the digital 3D model 103 may be continued, for example by scanning a larger surface and collecting larger amount of scan data compared to a suboptimal position.

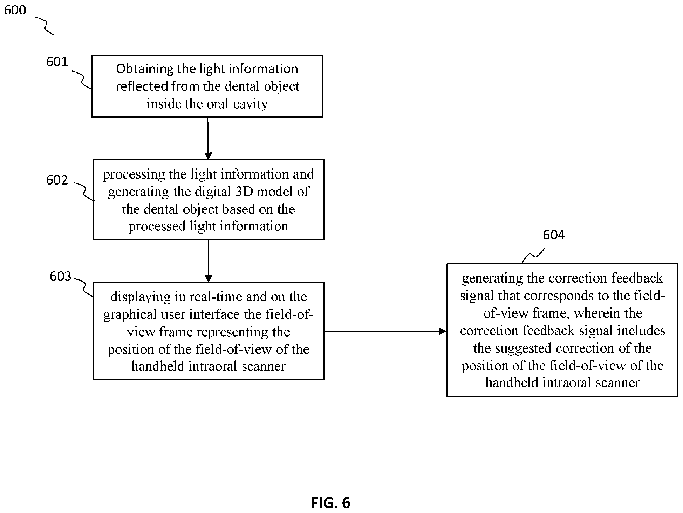

In figure 6, steps of a method 600 for generating the correction feedback signal 301 during the scan session are illustrated. The method 600 comprises obtaining 601 the light information reflected from the dental object 108 inside the oral cavity through the field-of-view 105 of the handheld intraoral scanner 101. Further, the method shows processing 602 the light information and generating the digital 3D model 103 of the dental object 108 based on the processed light information. Further, the method illustrates displaying 603 in real-time and on the graphical user interface 102, the field-of-view frame 104 representing the position of the field-of-view 105 of the handheld intraoral scanner 101. Further illustrated is the step of generating 604 the correction feedback signal 301 that corresponds to the field-of-view frame 104, wherein the correction feedback 301 signal includes the suggested correction of the position of the field-of-view 105 of the handheld intraoral scanner 101.

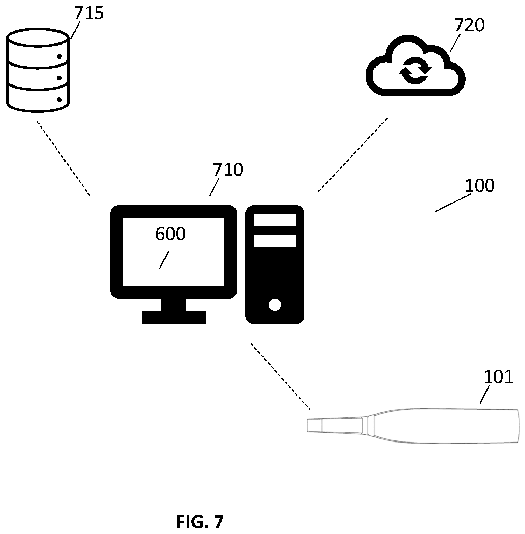

Figure 7 illustrates the intraoral scanner system 100 which may comprise a computer 710 capable of carrying out any method of the disclosure. The computer 710 may comprise a wired or a wireless interface to a server 715, a cloud server 720 and the wired or wireless handheld intraoral scanner 101. The handheld intraoral scanner 101 may be capable of obtaining light information reflected from the patient's oral cavity. The handheld intraoral scanner 101 may be equipped with various modules such as a fluorescence module, natural color module and/or an infrared module and thereby capable of capturing information relevant for diagnosing dental conditions such as caries, tooth cracks, gingivitis, dental recession and/or plaque.

The intraoral scanner system 100 may comprise a data processing device configured to carry out the method according to one or more embodiments of the disclosure. The data processing device may be a part of the computer 710, the server 715, the cloud server 720, or a handheld device not shown in the figure.

A non-transitory computer-readable storage medium may be comprised in the intraoral scanner system 100. The non-transitory computer-readable medium can carry instructions which, when executed by the computer, cause the computer to carry out the method according to one or more embodiments of the disclosure.

A computer program product may be embodied in the non-transitory computer-readable storage medium. The computer program product may comprise instructions which, when executed by the computer, cause the computer to perform the method according to any of the embodiments presented herein.

## Claims

1. An intraoral scanner system (100) configured to display a correction feedback signal (301) during a scan session, wherein the intraoral scanner system (100) comprises:
- a handheld intraoral scanner (101) configured to obtain light information reflected from a dental object (108) inside an oral cavity through a field-of-view (105) of the handheld intraoral scanner (101);
- one or more processors configured to process the light information and to generate a digital 3D model (103) of the dental object (108) based on the processed light information; and
- a graphical user interface (102) configured to display the digital 3D model (103),
wherein the one or more processors are configured to:
- display in real-time and on the graphical user interface (102) a field-of-view frame (104) representing a position of the field-of-view (105) of the handheld intraoral scanner (101); and
- generate a correction feedback signal (301) that corresponds to the field-of-view frame (104), and wherein the correction feedback signal (301) includes a suggested correction of the position of the field-of-view (105) of the handheld intraoral scanner (101).

2. The system (100) according to claim 1, further wherein the correction feedback signal (301) is **characterized by** a signal direction, wherein the signal direction is determined based on a relative position of a part of the digital 3D model (103) rendered in the field-of-view frame (104) with respect to the field-of-view frame (104).

3. The system (100) according to claim 1 or 2, wherein the one or more processors are configured to generate the correction feedback signal (301) when the handheld intraoral scanner (101) moves in a manner such that an amount of collected scan data is reduced.

4. The system (100) according to any previous claim, wherein the one or more processors are configured to generate the correction feedback signal (301) when a loss of registration of the obtained light information is detected.

5. The system (100) according to any previous claim, wherein the one or more processors are configured to generate the correction feedback signal (301) when the generation of the digital 3D model (103) is interrupted.

6. The system (100) according to any previous claim, wherein the one or more processors are configured to generate the correction feedback signal (301) when the generation of the digital 3D model (103) occurs in a suboptimal manner from less scan data compared to obtainable scan data.

7. The system (100) according to any previous claim, wherein the correction feedback signal (301) comprises a shape indicating the suggested correction of the position of the field-of-view (105) of the handheld intraoral scanner (101).

8. The system (100) according to any previous claim, wherein the correction feedback signal (301) comprises a dynamic pattern indicating the suggested correction of the position of the field-of-view (105) of the handheld intraoral scanner (101), wherein the dynamic pattern is a moving pattern of shapes.

9. The system (100) according to any previous claim, wherein the one or more processors are configured to:
- determine a deviation of the position of the field-of-view (105) of the handheld intraoral scanner (101) from a scan path (200); and
- determine the suggested correction of a position of the field-of-view (105) of the handheld intraoral scanner (101) based on the determined deviation and the current position of the field-of-view (105) of the handheld intraoral scanner (101).

10. The system (100) according to claim 9, wherein the scan path (200) is one of:
- a selectable scan path that is selected amongst several selectable scan paths for different dentitions; or
- a dynamic scan path that is configured to be modified during a scan session by the one or more processors to adapt relative to a current position of the field-of-view (105) of the handheld intraoral scanner (101).

11. The system (100) according to any of the previous claims, wherein the one or more processors are configured to:
- determine an area of the digital 3D model (103) comprising missing information;
- determine a direction and/or orientation of the field-of-view (105) of the handheld intraoral scanner (101) towards the area of the digital 3D model (103), and
- generate the suggested correction based on the determined direction and/or orientation.

12. The system (100) according to any previous claim, wherein the suggested correction is based on a prediction of a location of a neighboring dental object to the dental object being scanned.

13. A method for generating a correction feedback signal (301) during a scan session, wherein the method comprises:
- obtaining light information reflected from a dental object (108) inside an oral cavity through a field-of-view (105) of a handheld intraoral scanner (101),
- processing the light information and generating the digital 3D model (103) of the dental object (108) based on the processed light information,
- displaying in real-time and on a graphical user interface (102) a field-of-view frame (104) representing the position of the field-of-view (105) of the handheld intraoral scanner (101),
- generating the correction feedback signal (301) that corresponds to the field-of-view frame (104), wherein the correction feedback signal (301) includes the suggested correction of the position of the field-of-view (105) of the handheld intraoral scanner (101).

14. A computer program product comprising instructions which, when the program is executed by a computer, cause the computer to carry out the method of claim 13.

15. A computer-readable medium having stored thereon the computer program product of claim 14.
